# EUROPÄISCHE PATENTSCHRIFT

(11) **EP 1 162 185 B1**
(45) Veröffentlichungstag und Bekanntmachung des Hinweises auf die Patenterteilung: **01.10.2003**
(21) Anmeldenummer: 01113142.2
(22) Anmeldetag: 30.05.2001
(51) Int. Cl.: C07C 25/24, C09K 19/32

(54) **Hydrierte Phenanthrene und ihre Verwendung in Flüssigkristallmischungen**
Hydrogenated phenanthrenes and their use in liquid crystal mixtures
Phénanthrènes hydrogénés et leur utilisation dans des mélanges liquides cristallins

(30) Priorität: 08.06.2000 DE 10028451
(43) Veröffentlichungstag der Anmeldung: 12.12.2001
(73) Patentinhaber: MERCK PATENT GmbH, 64293 Darmstadt (DE)
(72) Erfinder: Wingen, Rainer, 65795 Hattersheim (DE); Hornung, Barbara, 63594 Hasselroth (DE); Schmidt, Wolfgang, 63303 Dreieich (DE)

(56) Entgegenhaltungen:
- DE-A- 19 748 819
- DATABASE WPI Section Ch, Week 200144 Derwent Publications Ltd., London, GB; Class E14, AN 2001-412128 XP002176961 & JP 2001 048823 A (DAINIPPON INK & CHEM INC), 20. Februar 2001 (2001-02-20)
- DATABASE WPI Section Ch, Week 200130 Derwent Publications Ltd., London, GB; Class E19, AN 2001-226501 XP002176962 & AU 56488 99 A (DAINIPPON INK & CHEM INC) , 5. März 2001 (2001-03-05)

## Beschreibung

Für die Verwendung in flüssigkristallinen Mischungen sind Derivate des Phenanthrens bereits aus DE-A 19500768, WO 98 / 27043, WO 98 /27035 oder WO 99/24385 bekannt.

Da aber die Entwicklung von Flüssigkristallmischungen noch in keiner Weise als abgeschlossen betrachtet werden kann, sind die Hersteller von Displays an den unterschiedlichsten Komponenten für Mischungen interessiert.

Insbesondere werden Flüssigkristallmischungen benötigt, die einerseits einen sehr weiten Arbeits-Temperaturbereich aufweisen, andererseits eine möglichst geringe Schwellspannung besitzen, beispielsweise für den Einsatz in Automobilen, in denen ohne weiteres ein Temperaturbereich von -40°C bis 100°C auftreten kann, aber auch für tragbare Geräte wie Mobiltelefone und Notebook-PCs..

Es besteht daher eine anhaltende Nachfrage nach neuen, geeigneten Flüssigkristallmischungen und -Mischungskomponenten.

Aufgabe der vorliegenden Erfindung ist es daher, neue Komponenten für die Verwendung in nematischen oder cholesterischen Flüssigkristallmischungen bereit zu stellen, die über positive Werte der dielektrischen Anisotropie kombiniert mit einem günstigen Verhältnis von Viskosität und Klärpunkt verfügen. Darüber hinaus sollen die Verbindungen in hohem Maße licht- und UVstabil sowie thermisch stabil sein. Ferner sollen sie geeignet sein, hohe ,voltage holding ratio (VHR)' zu realisieren. Ferner sollten sie synthetisch gut zugänglich und daher potentiell kostengünstig sein.

Es wurde nun gefunden, daß diese Anforderung erfüllt werden von den hydrierten Phenanthrenen der Formel (I) 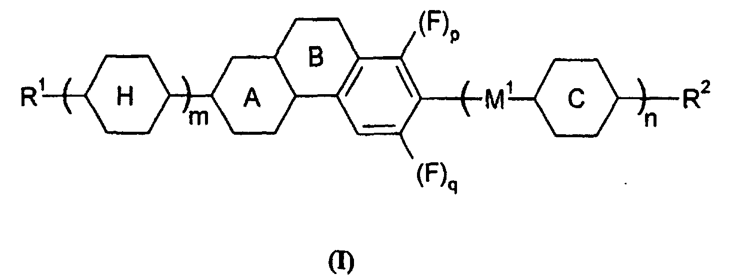 worin bedeuten:
- **R**^{**1**}: H, einen Alkylrest mit 1 bis 12 C-Atomen oder einen Alkenylrest mit 2 bis 8 C-Atomen, worin auch jeweils eine (nicht terminale) -CH₂-Gruppe durch -O- oder -C(=O)O- ersetzt sein kann und/oder ein oder mehrere H durch F ersetzt sein können,
- **R**^{**2**}: H, F, einen Alkylrest oder Alkyloxyrest mit 1 bis 12 C-Atomen oder einen Alkenylrest oder Alkenyloxyrest mit 2 bis 8 C-Atomen, worin auch jeweils eine (nicht terminale) -CH₂-Gruppe durch -O- oder -C(=O)O-ersetzt sein kann und/oder ein oder mehrere H durch F ersetzt sein können,
- **M**^{**1**}: -C(=O)O-, -OC(=O)-, -CH₂O-, -OCH₂-, -C=C-, -CH₂CH₂ oder eine Einfachbindung,
- **m, n**: unabhängig voneinander Null oder 1; m+n 0 oder 1,
- **p, q**: unabhängig voneinander Null oder 1; p+q 1 oder 2, 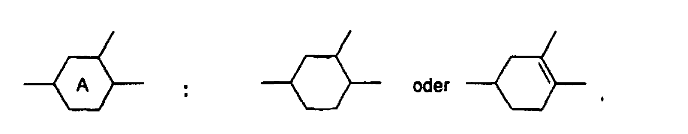 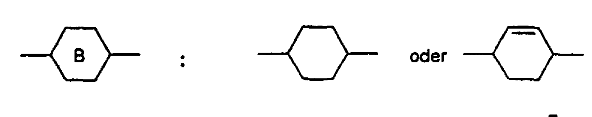
und 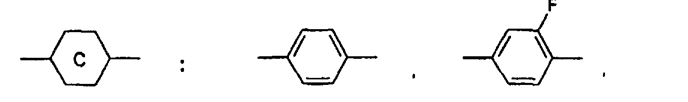 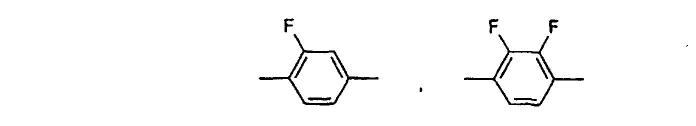 oder

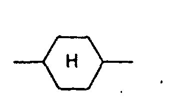

Bevorzugt sind die Verbindungen der Formeln (Ia) bis (Im):

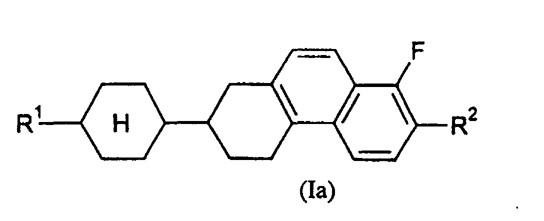

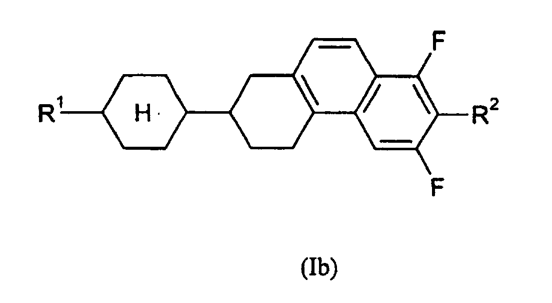

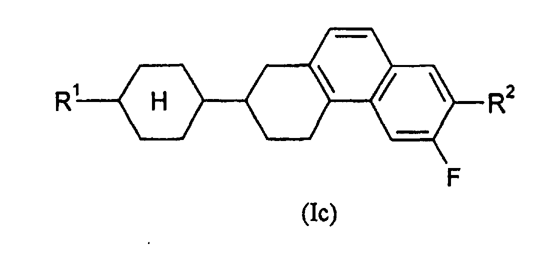

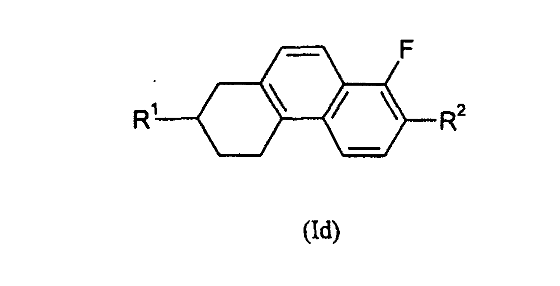

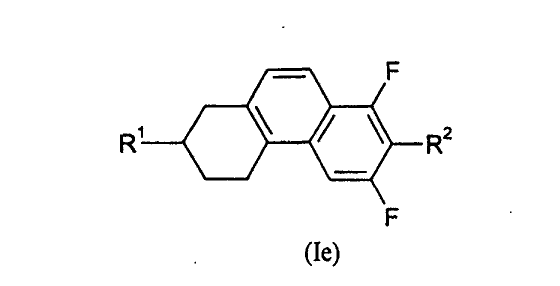

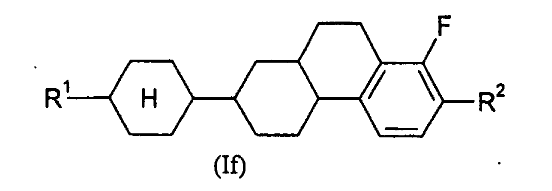

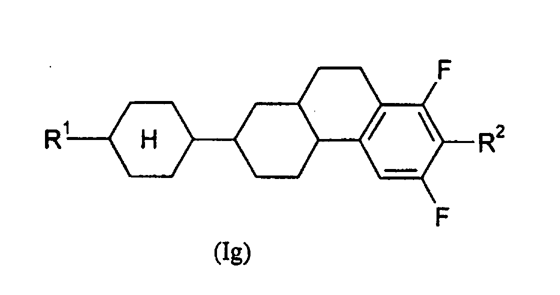

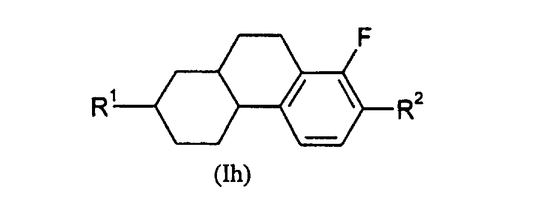

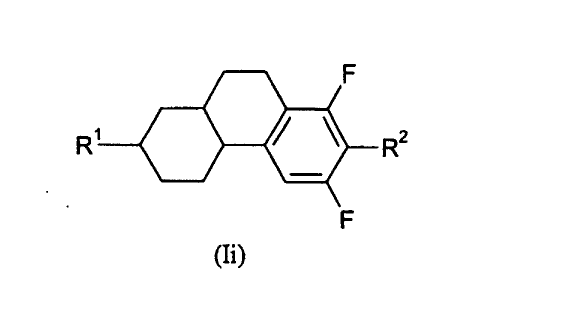

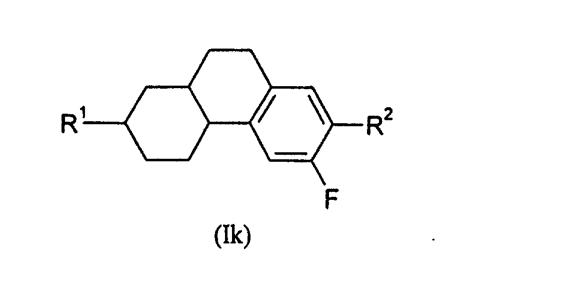

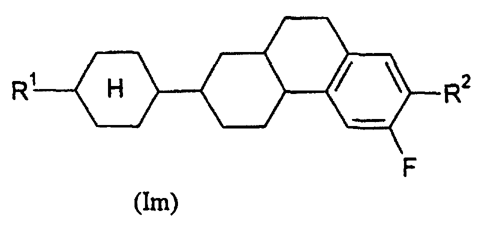

Bei der Bezeichnung der Verbindungen wurde auf die Buchstaben j und l verzichtet, da diese mit i bzw I verwechselt werden können.

Ganz besonders bevorzugt sind die Verbindungen der Formeln (Ia), (Ib), (Ie), (Ig), (Ih), (Ii) und (Ik), in denen R² bedeutet:
a) F,
b) einen Alkyl- oder Alkyloxy-Rest mit 1 bis 2 C-Atomen, bei dem ein oder mehrere H durch F ersetzt sind oder
c) einen Alkenyl- oder Alkenyloxy-Rest mit 2 C-Atomen, bei dem ein oder mehrere H durch F ersetzt sind.

Speziell bevorzugt sind die Verbindungen der Formeln (Ii1), (Ig1), (Ib1) und (Ie1)

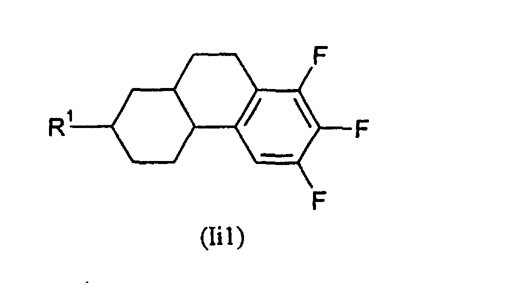

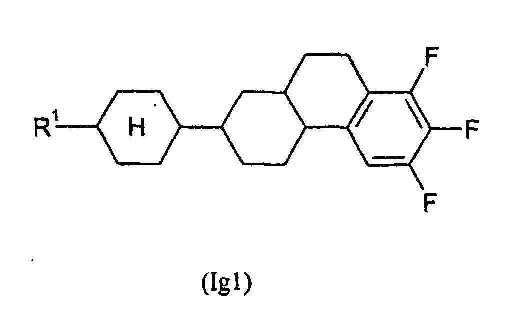

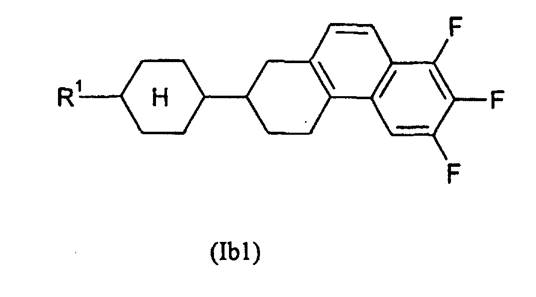

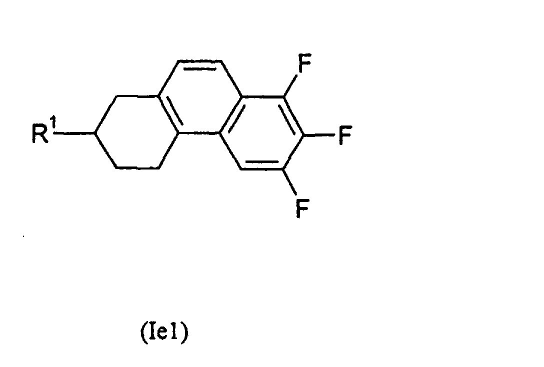

Die Verbindungen der Formel (I) werden in, vorzugsweise nematischen oder cholesterischen, Flüssigkristallmischungen eingesetzt. Die erfindungsgemäßen Flüssigkristallmischungen enthalten mindestens eine Verbindung der Formel (I), vorzugsweise in einer Menge von 1 bis 40 Gew.-%, bezogen auf die Flüssigkristallmischung. Vorzugsweise enthalten sie mindestens 3 weitere Komponenten. Die Erfindung betrifft auch ein Flüssigkristalldisplay, das diese Flüssigkristallmischungen enthält.

Die Erfindung wird durch die nachstehenden Beispiele näher erläutert.

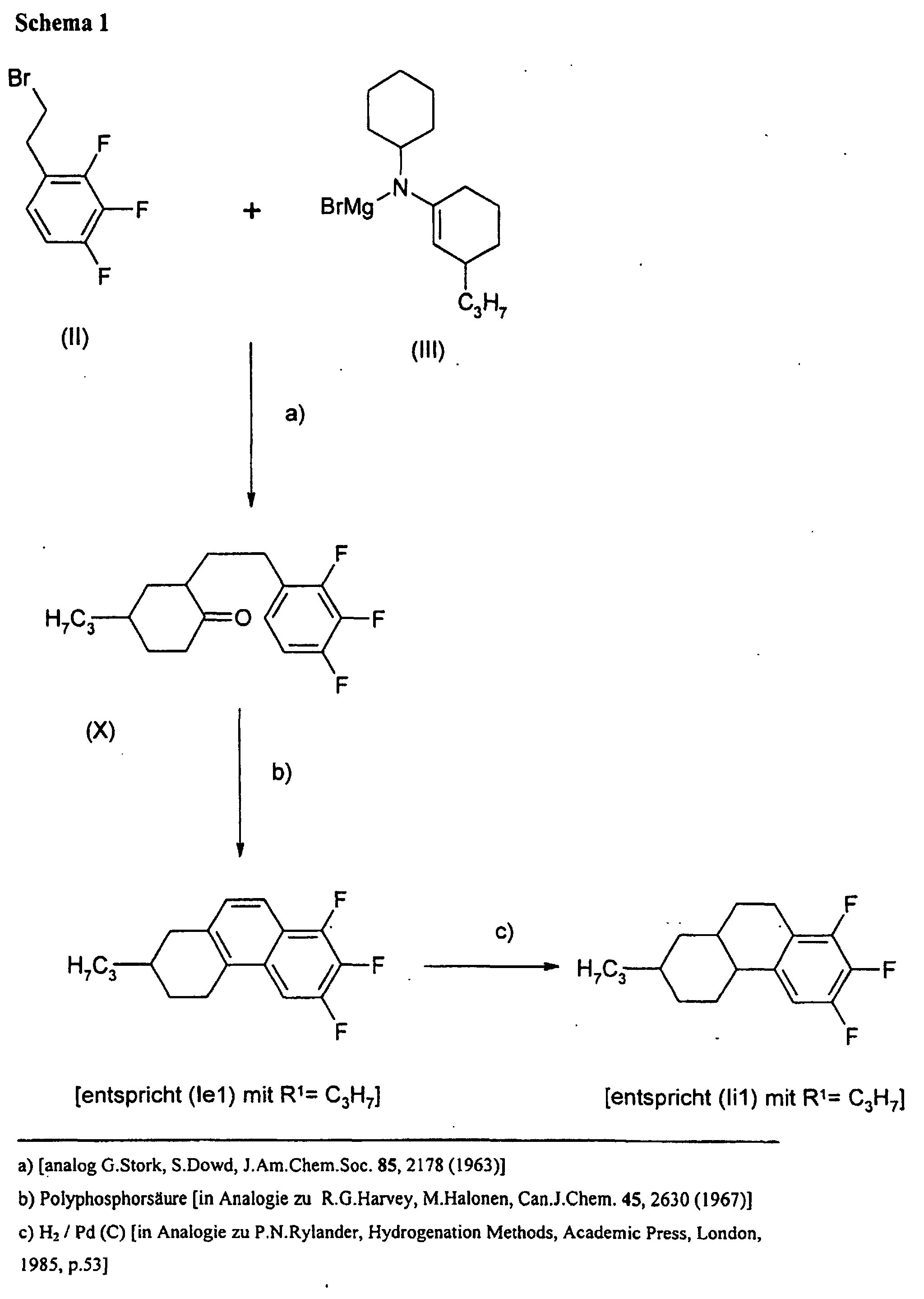

Die an einer konkreten Verbindung angegebene Synthese nach Schema 1 läßt sich durch Variation der Fluorverbindung (II) bzw. der Alkylketten der Cyclohexylaminöverbindung (III) bzw. Ersatz des Alkylsubstituenten in (III) durch einen 4-Alkylcyclohexylrest breit für die Synthese der erfindungsgemäßen Verbindungen anwenden.
So kann beispielsweise Ersatz von 2-(2,3,4-Trifluorphenyl)ethylbromid (hergestellt analog R.P.Houghton, M.Voyle, R.Price, J.Chem.Soc. Perkin Trans 1 (1984), 925) durch 2-(2,3-Difluorphenyl)ethylbromid [126163-29-9] zu Verbindungen der Formel (Id) bzwz. (Ih) führen, in denen R² F bedeutet; gleichermaßen können durch Einsatz von 2-(2-Fluorphenyl)ethylbromid [91319-54-9] als (II) Verbindungen der Formeln (Id) bzw. (Ih) erhalten werden, in denen R² = H ist. Hierauf lassen sich die weiter unten aufgeführten Folgereaktionen einer ortho-Metallierung anwenden.
Für die Synthese des Zwischenproduktes (X) stehen mit T.Cuvigny, H.Normant, Organometallics Chem.Synth., 1(1971) 237 bzw. T.Takeda, H.Taguchi, T.Fujiwara, Tetrahedron Lett. 41 (2000) 65 Verfahrensvarianten zur Verfügung.

Eine weitere Synthesemöglichkeit ist in Schema 2 aufgeführt, ebenfalls am Beispiel einer konkreten Verbindung. Dem Fachmann ist aber geläufig, daß Ersatz des fluorierten Tetralons (IV) [1101931-79-8] durch 7-Fluor-1-tetralon [2840-44-0] zu Verbindungen der Formel (Ik) bzw. (Im) führen wird, während bei Einsatz von 5-Fluor-1-tetralon [93742-85-9] Verbindungen der Formel (Ih) bzw. (If) erhalten werden können. Ebenfalls geläufig ist, daß durch Variation des Alkylhalogenids in der Reaktionsstufe d) eine Variation des Substituenten R¹ der erfindungsgemäßen Verbindungen erzielt werden kann.
Ausgehend von (V) kann der Fachmann nach Standardmethoden der ortho-Metallierung - z.B. mit "Schlosser-Base" - den Substituenten R² in Form einer Alkylgruppe einführen; alternativ kann die primär gebildete Lithiumverbindung mit Borsäureestern in die entsprechende Boronsäure und diese wiederum durch Oxidation in das entsprechende 2-Hydroxy-phenanthrenderivat überführt werden. Letzteres kann durch Williamson-Ethersynthese zu Verbindungen der Formel (Ii) [bzw. analog (Ig)] transformiert werden, in denen R² eine Alkyloxygruppe ist; durch Veresterung können Verbindungen der Formel (I) erhalten werden, in denen M¹ -OC(=O)- bedeutet.

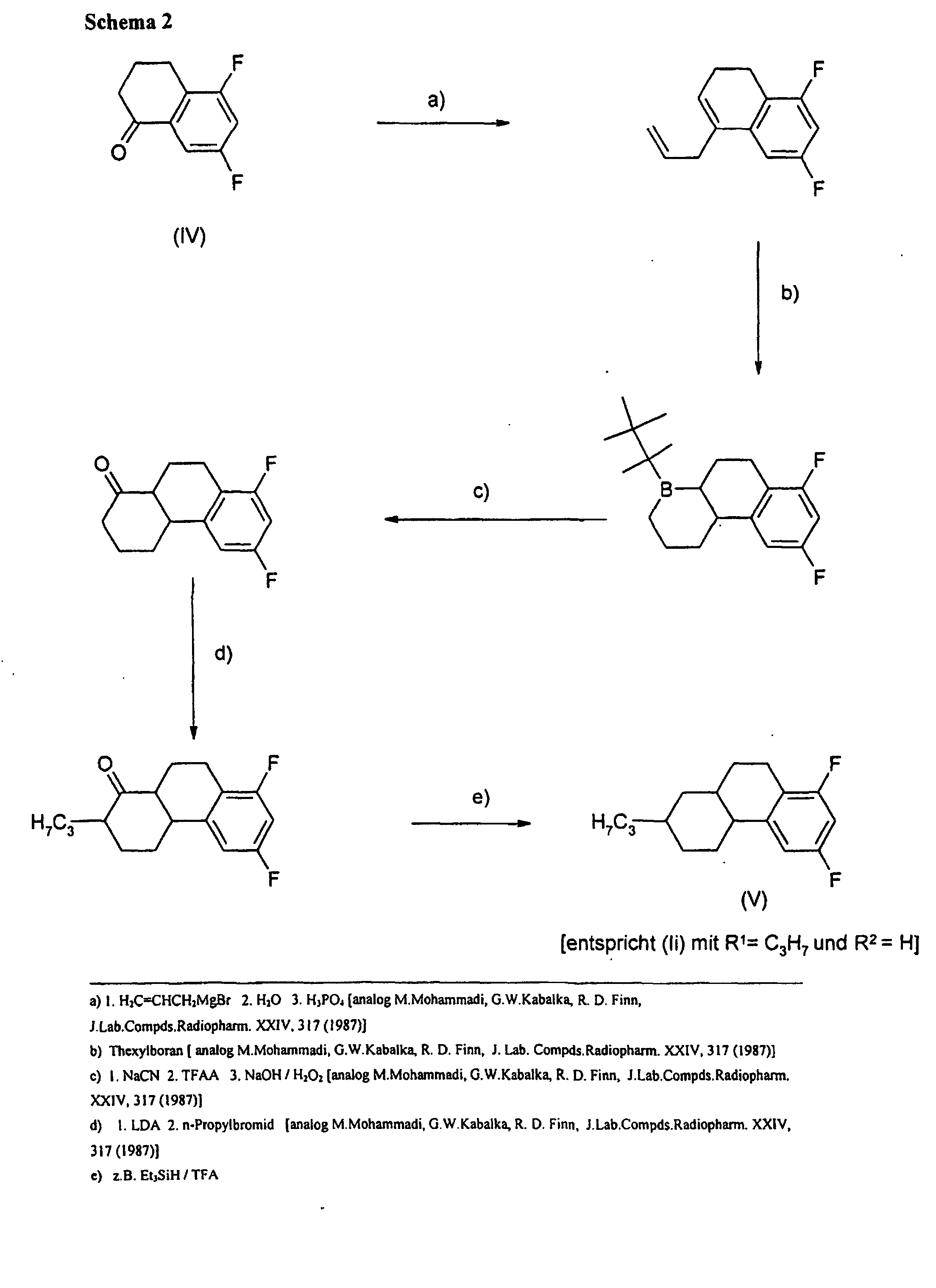

### Beispiel 1

### 6,8-Difluor-7-pentyl-2-propyl-1,2,3,4,4a,9,9a,10-octahydrophenanthren

Eine Lösung von 5,7-Difluor-1-tetralon [110931-79-8] (hergestellt nach DE-A 3702039) in Tetrahydrofuran wird bei 0°C zur äquimolaren Menge etherischer Allylmagnesiumbromid-Lösung gegeben. Nach üblicher wäßrig-saurer Aufarbeitung (Mohammadi et al., J.Lab.Cpd.Radiopharm. XXIV, 317 (1987)) und Destillation wird das erhaltene 5,7-Difluor-1-(1-propen-3-yl)-3,4-dihydronaphthalin mit einer äquimolaren Menge Thexylboran in Tetrahydrofuran zu B-Thexyl-6,8-difluor-2,3,4,4a,9,9a, 10-octahydro-1-bora-phenanthren umgesetzt, das ohne weitere Charakterisierung in THF-Lösung mit äquimolaren Mengen Natriumcyanid bei Raumtemperatur umgesetzt wird. Nach 2 h wird bei -75°C . tropfenweise mit einer äquimolaren Menge Trifluoressigsäureanhydrid (TFAA) versetzt und unter Rühren auf Raumtemperatur gebracht. Nach Zugabe von 3 Äquivalenten NaOH wird mit 50% H₂O₂, zuletzt bei 50°C, oxidiert; durch Extraktion und Chromatografie erhält man 1-Keto-6,8-difluor-1,2,3,4,4a,9,9a,10-octahydrophenanthren. Wie bei Mohammadi et al., J.Lab.Cpd.Radiopharm. XXIV, 317 (1987), beschrieben, läßt sich dieses Umsetzung mit Lithiumdiisoproylamid (LDA) mit anschließender Zugabe von n-Propylbromid in 1-Keto-6,8-difluor-2-propyl -1,2,3,4,4a,9,9a, 10-octahydrophenanthren überführen. Durch Reduktion mit Triethylsilan in Trifluoressigsäure läßt sich daraus 6,8-Difluor-1,2,3,4,4a,9,9a, 10-octahydrophenanthren erhalten. Durch ortho-Metallierung mit "Schlosser-Base" (d.h. Butyllithium und Kalium-tertbutylat; siehe z.B. F. Mongin, M. Schlosser, Tetrahedron Lett. 1996, 37, 6551-6554) bei -75°C und "Quenchen" mit n-Pentylbromid sowie üblicher Aufarbeitung durch Hydrolyse, Extraktion, Chromatografie ( Kieselgel, Dichlormethan) und mehrfache Umkristallisation (u.a. zur Anreicherung des gewünschten Isomeren) erhält man 6,8-Difluor-7-pentyl-2-propyl-1,2,3,4,4a,9,9a,10-octahydrophenanthren.

### Beispiel 2

Eine nematische Testmischung MLC-9000-100 (Hersteller: Merck KGaA, Darmstadt) wird mit 5% der Verbindung aus Bspl. 1 versetzt; man erhält folgende Verbesserungen gegenüber den in Klammern benannten Vergleichswerten der Mischung MLC-9000-100:
Klärpunkt (T(N,I)) = 92,5°C (90.5°C)
Rotationsviskosität (γ₁) = 196 mPas (201 mPas)
Doppelbrechung (Δn) = 0.1105 (0.1137).

### Beispiele 3 bis 28

Wie in Schema 1 gezeigt werden die folgenden Verbindungen hergestellt. Diese haben die in der folgenden Tabelle gezeigten Eigenschaften.

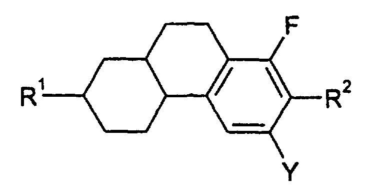

| Nr. | R¹ | Y | R² | Δn | Δε |
|---|---|---|---|---|---|
| 3 | CH₃ | H | F | | |
| 4 | C₂H₅ | H | F | | |
| 5 | *n*-C₃H₇ | H | F | | |
| 6 | *n*-C₄H₉ | H | F | | |
| 7 | *n*-C₅H₁₁ | H | F | | |
| 8 | *n*-C₆H₁₃ | H | F | | |
| 9 | *n*-C₇H₁₅ | H | F | | |
| 10 | CH₃O | H | F | | |
| 11 | C₂H₅O | H | F | | |
| 12 | *n*-C₃H₇O | H | F | | |
| 13 | CH₂=CH | H | F | | |
| 14 | CH₃-CH=CH | H | F | | |
| 15 | CH₃ | F | F | | |
| 16 | C₂H₅ | F | F | | |
| 17 | *n*-C₃H₇ | F | F | 0,081 | 14,6 |
| 18 | *n*-C₄H₉ | F | F | | |
| 19 | *n*-C₅H₁₁ | F | F | | |
| 20 | *n*-C₆H₁₃ | F | F | | |
| 21 | *n*-C₇H₁₅ | F | F | | |
| 22 | CH₃O | F | F | | |
| 23 | C₂H₅O | F | F | | |
| 24 | *n*-C₃H₇O | F | F | | |
| 25 | CH₂=CH | F | F | | |
| 26 | CH₃-CH=CH | F | F | | |
| 27 | *n*-C₃H₇ | H | OCF₃ | | |
| 28 | *n*-C₃H₇ | F | OCF₃ | | |

### Beispiele 29 bis 54

Wie in Schema 1 gezeigt werden die folgenden Verbindungen hergestellt. Diese haben die in der folgenden Tabelle gezeigten Eigenschaften.

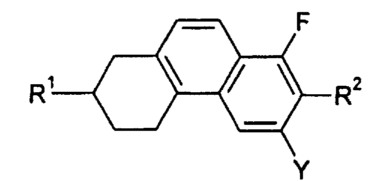

| Nr. | R¹ | Y | R² | Δn | Δε |
|---|---|---|---|---|---|
| 29 | CH₃ | H | F | | |
| 30 | C₂H₅ | H | F | | |
| 31 | *n*-C₃H₇ | H | F | | |
| 32 | *n*-C₄H₉ | H | F | | |
| 33 | *n*-C₅H₁₁ | H | F | | |
| 34 | *n*-C₆H₁₃ | H | F | | |
| 35 | *n*-C₇H₁₅ | H | F | | |
| 36 | CH₃O | H | F | | |
| 37 | C₂H₅O | H | F | | |
| 38 | *n*-C₃H₇O | H | F | | |
| 39 | CH₂=CH | H | F | | |
| 40 | CH₃-CH=CH | H | F | | |
| 41 | CH₃ | F | F | | |
| 42 | C₂H₅ | F | F | | |
| 43 | *n*-C₃H₇ | F | F | | |
| 44 | *n*-C₄H₉ | F | F | | |
| 45 | *n*-C₅H₁₁ | F | F | 0,139 | 17,3 |
| 46 | *n*-C₆H₁₃ | F | F | | |
| 47 | *n*-C₇H₁₅ | F | F | | |
| 48 | CH₃O | F | F | | |
| 49 | C₂H₅O | F | F | | |
| 50 | *n*-C₃H₇O | F | F | | |
| 51 | CH₂=CH | F | F | | |
| 52 | CH₃-CH=CH | F | F | | |
| 53 | *n*-C₃H₇ | H | OCF₃ | | |
| 54 | *n*-C₃H₇ | F | OCF₃ | | |

### Beispiele 55 bis 83

Analog zu Beispiel 3 werden die folgenden Verbindungen hergestellt. Diese haben die in der folgenden Tabelle gezeigten Eigenschaften.

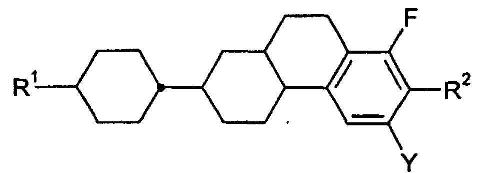

| Nr. | R¹ | Y | R² | Δn | Δε |
|---|---|---|---|---|---|
| 55 | CH₃ | H | F | | |
| 56 | C₂H₅ | H | F | | |
| 57 | *n*-C₃H₇ | H | F | | |
| 58 | *n*-C₄H₉ | H | F | | |
| 59 | *n*-C₅H₁₁ | H | F | | |
| 60 | *n*-C₆H₁₃ | H | F | | |
| 61 | *n*-C₇H₁₅ | H | F | | |
| 62 | CH₃O | H | F | | |
| 63 | C₂H₅O | H | F | | |
| 64 | *n*-C₃H₇O | H | F | | |
| 65 | CH₂=CH | H | F | | |
| 66 | CH₃-CH=CH | H | F | | |
| 67 | CH₃ | F | F | | |
| 68 | C₂H₅ | F | F | | |
| 69 | *n*-C₃H₇ | F | F | | |
| 70 | *n*-C₄H₉ | F | F | | |
| 71 | *n*-C₅H₁₁ | F | F | 0,081 | 11,0 |
| 72 | *n*-C₆H₁₃ | F | F | | |
| 73 | *n*-C₇H₁₅ | F | F | | |
| 74 | CH₃O | F | F | | |
| 78 | C₂H₅O | F | F | | |
| 79 | *n*-C₃H₇O | F | F | | |
| 80 | CH₂=CH | F | F | | |
| 81 | CH₃-CH=CH | F | F | | |
| 82 | *n*-C₃H₇ | H | OCF₃ | | |
| 83 | *n*-C₃H₇ | F | OCF₃ | | |

### Beispiele 84 bis 109

Analog zu Beispiel 29 werden die folgenden Verbindungen hergestellt. Diese haben die in der folgenden Tabelle gezeigten Eigenschaften.

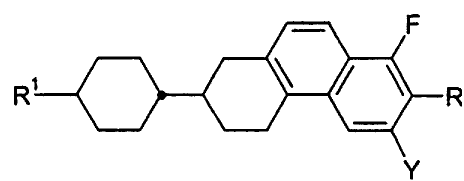

| Nr. | R¹ | Y | R² | Δn | Δε |
|---|---|---|---|---|---|
| 84 | CH₃ | H | F | | |
| 85 | C₂H₅ | H | F | | |
| 86 | *n*-C₃H₇ | H | F | | |
| 87 | *n*-C₄H₉ | H | F | | |
| 88 | *n*-C₅H₁₁ | H | F | | |
| 89 | *n*-C₆H₁₃ | H | F | | |
| 90 | *n*-C₇H₁₅ | H | F | | |
| 91 | CH₃O | H | F | | |
| 92 | C₂H₅O | H | F | | |
| 93 | *n*-C₃H₇O | H | F | | |
| 94 | CH₂=CH | H | F | | |
| 95 | CH₃-CH=CH | H | F | | |
| 96 | CH₃ | F | F | | |
| 97 | C₂H₅ | F | F | | |
| 98 | *n*-C₃H₇ | F | F | 0,132 | 15,2 |
| 99 | *n*-C₄H₉ | F | F | | |
| 100 | *n*-C₅H₁₁ | F | F | | |
| 101 | *n*-C₆H₁₃ | F | F | | |
| 102 | *n*-C₇H₁₅ | F | F | | |
| 103 | CH₃O | F | F | | |
| 104 | C₂H₅O | F | F | | |
| 105 | *n*-C₃H₇O | F | F | | |
| 106 | CH₂=CH | F | F | | |
| 107 | CH₃-CH=CH | F | F | | |
| 108 | *n*-C₃H₇ | H | OCF₃ | | |
| 109 | *n*-C₃H₇ | F | OCF₃ | | |

Sofern nicht jeweils explizit anders angegeben, gelten in der vorliegenden Anmeldung die folgenden Bedingungen. Die physikalischen Eigenschaften wurden bestimmt wie in Merck Liquid Crystals, Physical Properties of Liquid Crystals, Description of the Measurement Methods, Hrg. W. Becker (Nov. 1997) beschrieben. Alle Konzentrationsangaben sind Massenprozente, alle Temperaturen Grad Celsius und alle Temperaturdifferenzen Differenzgrad Celsius. Alle physikalischen Eigenschaften gelten für 20 °C, die Brechungsindicees gelten für eine Wellenlänge von 589 nm, und die dielektrischen Anisotropien für eine Frequenz von 1 kHz.

## Patentansprüche

1. Hydrierte Phenanthrenderivate der Formel (I) 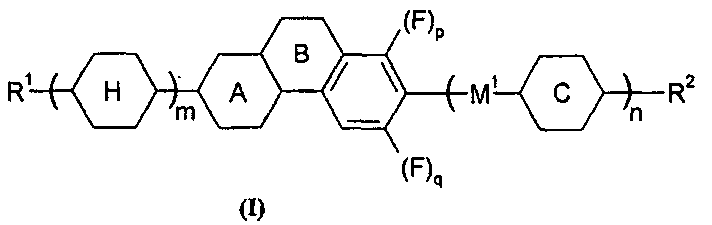 worin bedeuten:
**R**^{**1**} H, einen Alkylrest mit 1 bis 12 C-Atomen oder einen Alkenylrest mit 2 bis 8 C-Atomen, worin auch jeweils eine (nicht terminale) -CH₂-Gruppe durch -O- oder -C(=O)O- ersetzt sein kann und/oder ein oder mehrere H durch F ersetzt sein können,
**R**^{**2**} H, F, einen Alkylrest oder Alkyloxyrest mit 1 bis 12 C-Atomen oder einen Alkenylrest oder Alkenyloxyrest mit 2 bis 8 C-Atomen, worin auch jeweils eine (nicht terminale) -CH₂-Gruppe durch -O- oder -C(=O)O-ersetzt sein kann und/oder ein oder mehrere H durch F ersetzt sein können,
**M**^{**1**} -C(=O)O-, -OC(=O)-, -CH₂O-, -OCH₂-, -C=C-, -CH₂CH₂ oder eine Einfachbindung,
**m, n** unabhängig voneinander Null oder 1; m+n 0 oder 1,
**p, q** unabhängig voneinander Null oder 1; p+q 1 oder 2, 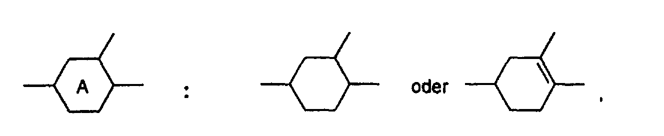 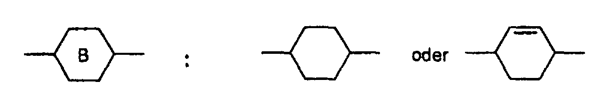
und 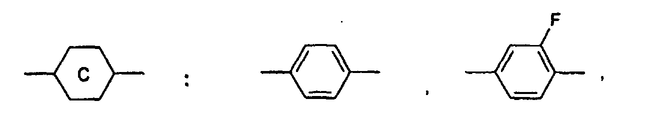 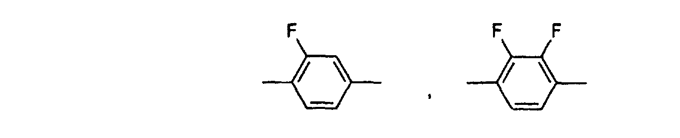 oder

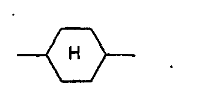

2. Verbindungen nach Anspruch 1, **dadurch gekennzeichnet, daß** in der Formel (I) n = 0 ist, 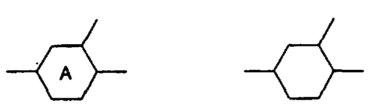 und 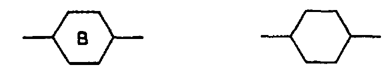 bedeutet.

3. Verbindungen nach Anspruch 1, **dadurch gekennzeichnet, daß** in der Formel (I) n = 0 ist, 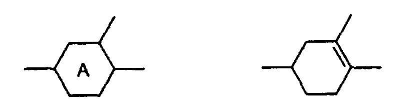 und 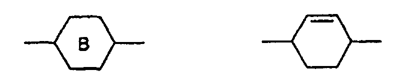 bedeutet.

4. Verbindungen ausgewählt aus der Gruppe der Verbindungen der Formeln If bis Im 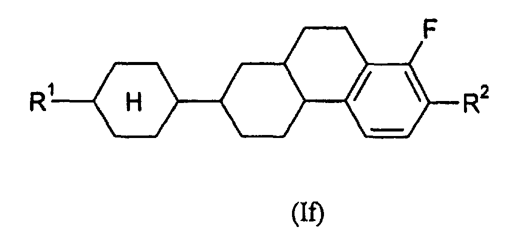 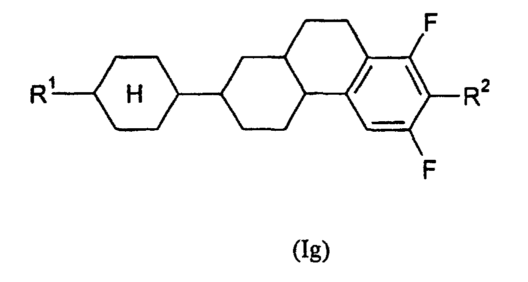 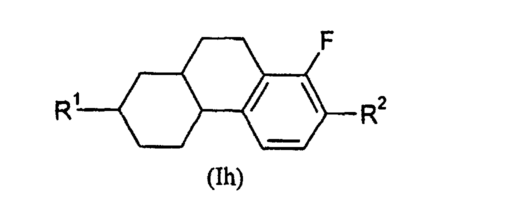 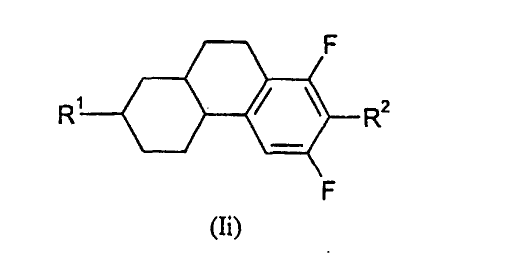 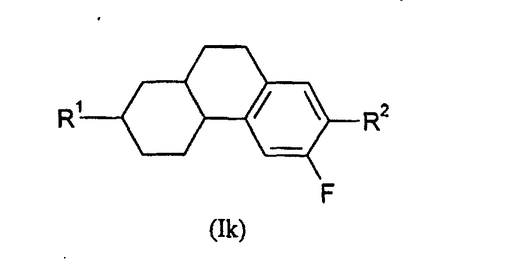 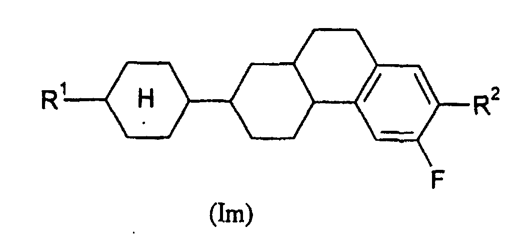 in denen R² bedeutet:
a) F,
b) einen Alkyl- oder Alkyloxy-Rest mit 1 bis 2 C-Atomen, bei dem ein oder mehrere H durch F ersetzt sind oder
c) einen Alkenyl- oder Alkenyloxy-Rest mit 2 C-Atomen, bei dem ein oder mehrere H durch F ersetzt sind.

5. Verwendung von Verbindungen der Formel (I) gemäß mindestens einem der Ansprüche 1 bis 4 in Flüssigkristallmischungen.

6. Flüssigkristallmischung , **dadurch gekennzeichnet, daß** sie die Verbindung(en) gemäß mindestens einem der Ansprüche 1 bis 4 in einer Menge von 1 bis 40 Gew.-%, bezogen auf die Flüssigkristallmischung enthält.

7. Flüssigkristallmischung nach Anspruch 6, **dadurch gekennzeichnet, daß** sie nematisch oder cholesterisch ist.

8. Flüssigkristalldisplay, enthaltend eine Flüssigkristallmischung nach mindestens einem der Ansprüche 6 und 7.

## Claims

1. Hydrogenated phenanthrene derivatives of the formula (I) 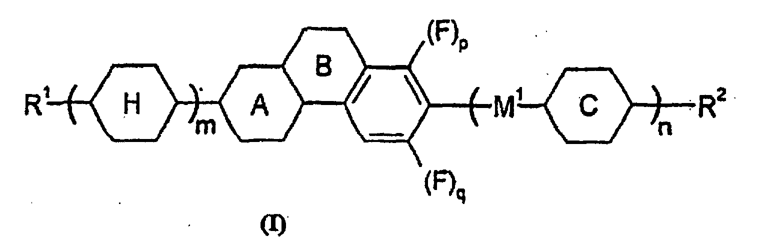 in which:
**R**^{**1**} is H, an alkyl radical having from 1 to 12 carbon atoms or an alkenyl radical having from 2 to 8 carbon atoms, in each of which a (non-terminal) -CH₂- group may be replaced by -O- or -C(=O)O- and/or one or more H may be replaced by F,
**R**^{**2**} is H, F, an alkyl radical or alkoxy radical having from 1 to 12 carbon atoms or an alkenyl radical or alkenyloxy radical having from 2 to 8 carbon atoms, in each of which a (non-terminal) -CH₂- group may be replaced by -O- or -C(=O)O- and/or one or more H may be replaced by F,
**M**^{**1**} is -C(=O)O-, -OC(=O)-, -CH₂O-, -OCH₂-, -C≡C-, -CH₂CH₂- or a single bond,
**m and n,** independently of one another, are zero or 1; m + n is 0 or 1,
**p and q,** independently of one another, are zero or 1; p + q is 1 or 2, 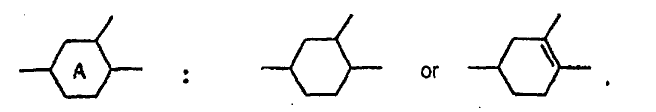 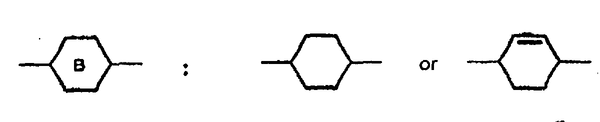
and 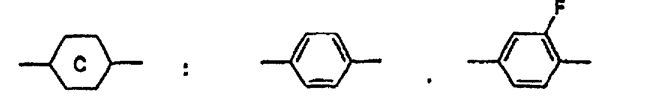 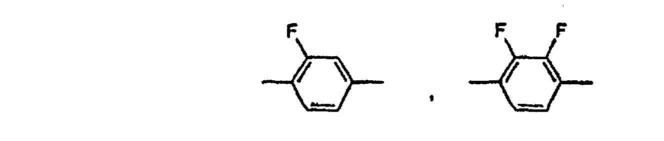 or

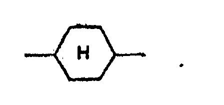

2. Compounds according to Claim 1, **characterised in that**, in the formula (I), n = 0, 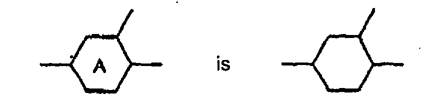 and

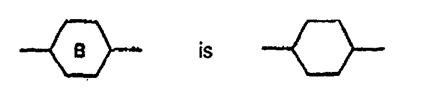

3. Compounds according to Claim 1, **characterised in that**, in the formula (I), n = 0, 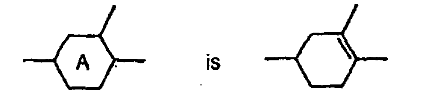 and

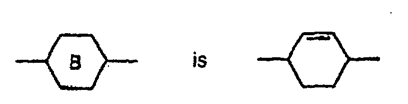

4. Compounds selected from the group of compounds of the formulae If to Im 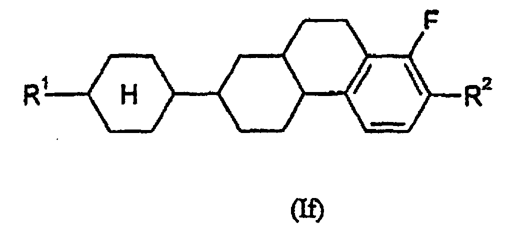 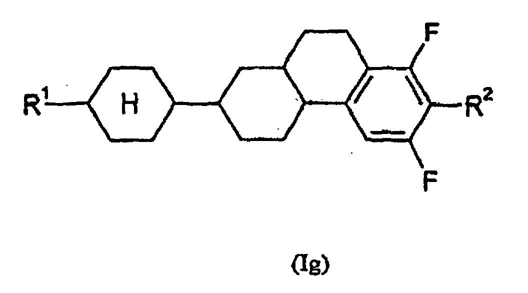 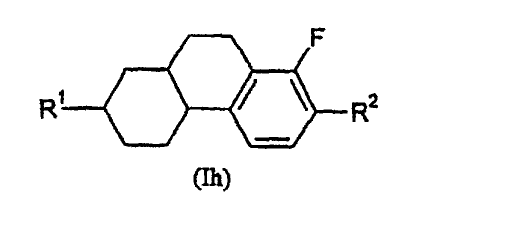 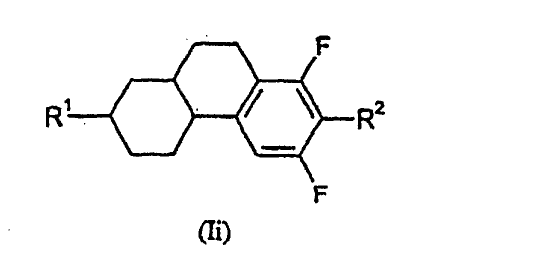 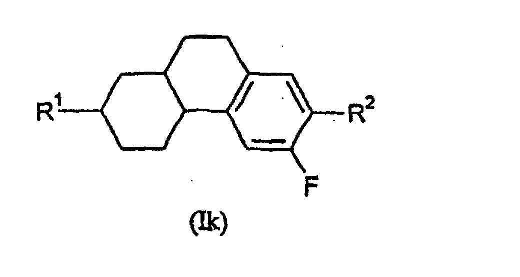 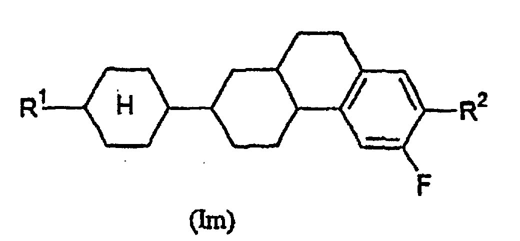 in which R² is:
a) F,
b) an alkyl or alkoxy radical having 1 or 2 carbon atoms, in which one ore more H may be replaced by F, or
c) an alkenyl or alkenyloxy radical having 2 carbon atoms, in which one ore more H may be replaced by F.

5. Use of compounds of the formula (I) according to at least one of Claims 1 to 4 in liquid-crystal mixtures.

6. Liquid-crystal mixture, **characterised in that** it comprises the compound(s) according to at least one of Claims 1 to 4 in an amount of from 1 to 40% by weight, based on the liquid-crystal mixture.

7. Liquid-crystal mixture according to Claim 6, **characterised in that** it is nematic or cholesteric.

8. Liquid-crystal display containing a liquid-crystal mixture according to at least one of Claims 6 and 7.

## Revendications

1. Dérivés hydrogénés de phénanthrène de lunaule (I) 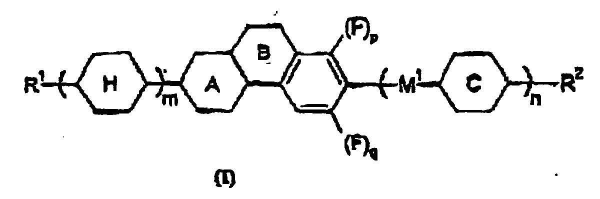 où
R¹ représente H, un radical alkyle ayant de 1 à 12 atomes de C ou un radical alcènyle ayant 2 à 8 atomes de C, un groupe -CH₂ (non terminal) pouvant aussi respectivement être remplacé par -O- ou -C(=O)O- et/ou un ou plusieurs H pouvant être remplacés par F,
R² représente H, F, un radical alkyle ou un radical alkyloxy ayant de 1 à 12 atomes de C ou un radical alcènyle ou un radical alcènyloxy ayant de 2 à 8 atomes de C, un groupe -CH₂ (non terminal) pouvant aussi respectivement être remplacé par -O- ou -C(=O)O-et/ou un ou plusieurs H pouvant être remplacés .par F,
M¹ représente -C (-O) O-, -OC (=O) -, -CH₂O-, -OCH₂-, -C≡C, -CH₂CH₂ ou une simple liaison,
m, n représentent, indépendamment l'un de l'autre, zéro ou 1 ; m+n égal 0 où 1,
p, q représentent, indépendamment l'un de l'autre, zéro ou 1 ; p+q égal 1 ou 2, 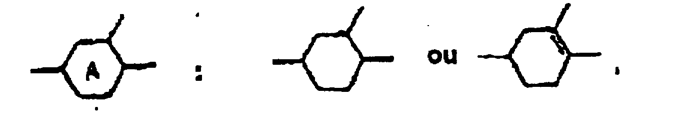 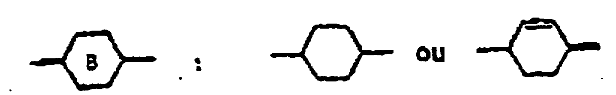 et 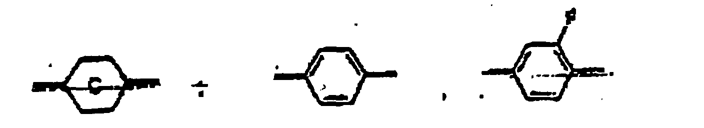 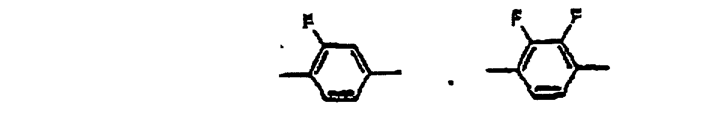 ou

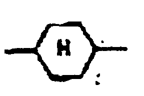

2. Composés selon la revendication 1, **caractérisés en ce que**, dans la formule (I), n = 0, 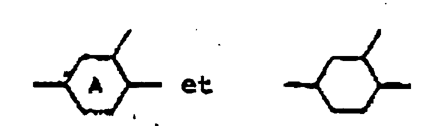 représentent

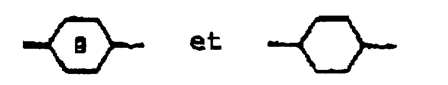

3. Composés selon la revendication 1, **caractérisés en ce que**, dans la formule (T), n = O, 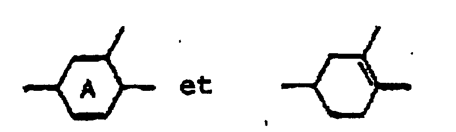 représentent

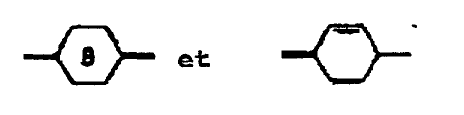

4. Composés choisis parmi le groupe des composés de formule If à Im 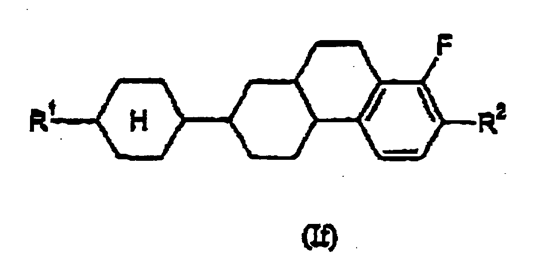 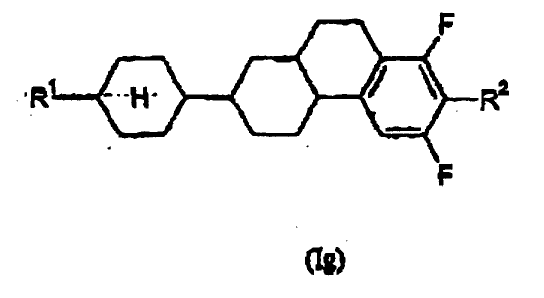 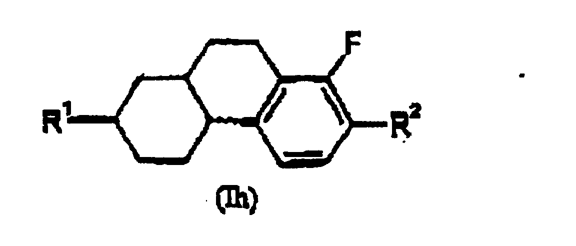 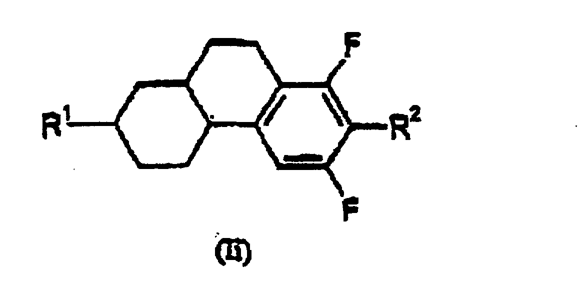 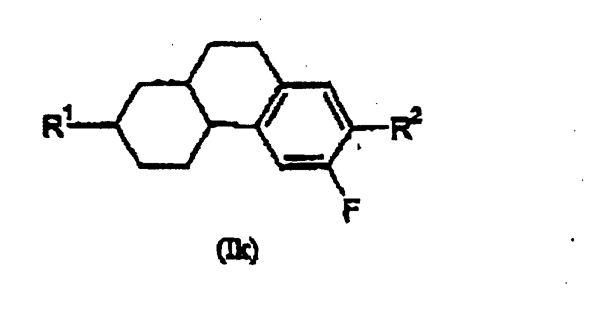 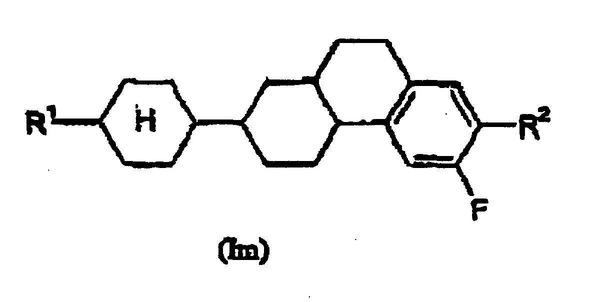 dans lesquelles R² représente
a) F,
b) un radical alkyle ou alkyloxy ayant de 1 à 2 atomes de C, dans lequel un où plusieurs H sont remplacés par F ou
c) un radical alcènyle ou alcènyloxy ayant 2 atomes de C dans lequel un ou plusieurs H sont remplacés par F.

5. Utilisation des composés de formule (I) selon au moins l'une quelconque des revendications 1 à 4 dans des mélanges de cristaux liquides.

6. Mélange de cristaux liquides, **caractérisé en ce qu'**il contient les composés selon au moins l'une quelconque des revendications 1 à 4 en une quantité allant de 1 à 40 % en poids rapporté sur le mélange de cristaux liquides.

7. Mélange de cristaux liquides selon la revendication 6, **caractérisé en ce qu'**il est nématique ou cholestérique.

8. Affichage aux cristaux liquides contenant un mélange de cristaux liquides selon au moins l'une quelconque des revendications 6 et 7.
